# EUROPEAN PATENT APPLICATION

(11) **EP 1 048 293 A1**
(43) Date of publication of application: **02.11.2000**
(21) Application number: 99303156.6
(22) Date of filing: 23.04.1999
(51) Int. Cl.: A61K 7/48

(54) **Headlice treatment compositions**

(71) Applicant: THE SCHOOL OF PHARMACY, UNIVERSITY OF LONDON, London WC1N 1AX (GB)
(72) Inventor: Williamson, Elizabeth Mary, London,WC1n 1ax (GB); Priestley, Caroline Mary, London,WC1N 1AX (GB)
(74) Representative: Jones, Helen Marjorie Meredith

(57) **Abstract**

Compositions for killing headlice and eggs are based on essential oils and/or terpenoids. The compositions contain high levels of terpinene-4-ol which is found to have high activity against both adult life and eggs.

## Description

The present invention relates to compositions useful for the treatment of headlice infestation. The compositions kill both the adult lice and the eggs. The compositions are based on essential oils, components thereof and mixtures.

The problem of headlice is persistent in developing and developed countries and there are many reasons why control programs have failed to reduce the incidents sufficiently. The nature of these reasons and their contribution to the lack of success not only vary between countries and continents but also more locally: in England, the contribution of resistance to pediculicides is different depending on the county.

The lack of application of theories about the development and spread of resistance to practical insecticide control measures has resulted in the development of resistance to almost all the currently available active compounds. In the future, the introduction of new chemical entities as pediculicides on the assumption that all headlice would be universally susceptible to a novel structure may help to combat current resurgence. These new molecules would continue to be useful provided that their use was properly managed, to prevent the lice from evolving to cope with them.

Recently, there have been concerns over the safety of currently available pediculicides. Media attention and exaggeration of the potential toxicity of specific, topically applied insecticides has left a feeling wariness towards them in the consumer. This may lead to non-compliance, either by refusal to use treatments, or application of the treatment in a lower frequency than suggested.

There is a particular type of medicine, however, which is gaining consumer confidence. Medicines containing natural products are generally regarded in the public eye as being safe, good and having fewer side effects, as well as providing a desired therapeutic effect.

For many commercial pediculicides, one failing has been the lack of complete activity against louse eggs. The structure of the egg is such that it is difficult to design a therapeutic product which will successfully penetrate the outer shell, besides being active in killing the developing louse inside. In the early stage of development inside the egg, the louse has no nervous system which is the target of most pediculicides.

Terpenoids have been known from ancient times to be lethal and repellent to insects and are considered to be an ovicidal component of some headlice formulations (Burgess, I. (1995) Adv. Parasitol. 36: 271-343). Although the successful use of essential oils in headlice infestations is known, few scientific studies have been carried out. Veal, L (1996) Comp. Ther. Nurs. Midwif. 2: 79-101 describes a study in which several essential oils and mixtures are tested for their pediculicidal properties. One of the oils tested is tea tree oil. The composition of the tea tree oil tested was not given. The composition of tea tree oil depends upon the source, in respect of the component terpenoids.

Two of the components of tea tree oil are 1,8-cineole and terpinene-4-ol. It is known that there is an inverse relationship between the content of 1,8-cineole and terpinene-4-ol. As the content of 1,8-cineole increases, the content of terpinene-4-ol decreases.

Terpinene-4-ol is described as an active ingredient in a insect repellent for clothes in JP-A-06-227906. The composition is in the form of a powder comprising granular active alumina containing the terpinine-4-ol.

In EP-A-0495684, lice repellent compositions comprise terpenoids such as terpinene, terpineol pulegone, perillyl alcohol, or isopulegone, are used on the hair and skin to repel lice.

In JP-A-04-108707, an insect repellent for repelling mosquitoes, black flies, midge, stable flies and cockroach, contains Japanese cypress essential oil.

In EP-A-0461134, a pediculicidal composition effective against adult lice and lice eggs comprises piperonal and a terpene to enhance the penetration of the composition into lice eggs.

In J.Pharm.Pharmacol. 49, suppl. 4 (1997), 120 Weston *et al* test terpenoid components of essential oils known to have pediculicidal properties for their lice killing activities, *in vitro* and *in vivo*.

One of the inventors of the present invention has formulated and publicly used lotions for treatment of headlice comprising the following components: formulation 1: oil of rosemary 1.25%, oil of lavender 1.25%, oil of geranium 1%, oil of juniper 1%, oil of eucalyptus 0.75% in a carrier of arachis oil; formulation 2: oil of lavender 1.25%, oil of geranium 1.25%, oil of juniper 1.25%, oil of eucalyptus 1.25% and oil of lemon 1.25% in a carrier of arachis oil; formulation 3: as formulation 2 but without lemon oil; formulation 4: 1.5% oil of eucalyptus 1.5% oil of juniper and 2% oil of lavender in a carrier of arachis oil.

A new composition according to the present invention for application direct to the skin or hair of a human for the treatment or prophylaxis of headlice infection comprises a carrier and a mixture of actives, in which the mixture of actives comprises at least 20% by volume terpinene-4-ol (T4ol), no more than 5% 1,8-cineole and contains at least 35% by volume components selected from T4ol thymol, alpha-terpineol, linalool and carveol.

In the composition, the ratio of T4ol to cineole is at least 4:1 preferably at least 5:1, for instance 10:1 or more, most preferably at least 15:1.

It is preferred that the composition contain low levels, for instance no more than 5% of the following compound: borneol, camphor, perillylketone, pulegone, thujone, safrol and estragole. Preferably such components, in combination, are present in a total amount of less than 5% by volume of the mixture of actives.

The terpenoids, which form the major proportion of the mixture of actives, may be synthetic, or derived from natural ingredients by chemical modification. Preferably, however, they are extracted from natural sources. Whilst the components may be substantially isolated, it is preferred for extracts comprising mixtures of terpenoids to be used in the compositions. The mixtures are essential oils, for instance extracted using steam distillation and/or solvent extraction techniques.

In the invention it is most preferred for the mixture of actives to comprise a blend of two or more essential oils. Often at least three essential oils will be used. The essential oils are selected having regard to their content of T4ol, which the present inventors have established has high pediculicidal and ovicidal properties, as well their content of the other terpenoids specified in the claims. Essential oils used in the present invention are preferably selected from the following oils: tea tree, especially meeting ISO 4730 standard, for instance extracted from *Malalaleuca alternifolia*; thyme oil (especially thyme oil composed of at least 20% by volume thymol); peppermint oil (especially oil comprising at least 20% by volume methone); lavender oil (especially comprising at least 20% by volume linalool); cabreuva oil or cubeb seed (preferably containing at least 20% by volume nerolidol); pine oil (preferably comprising at least 20% by volume alpha-terpineol); oil of ajowan (*Trachyspermum ammi*) (preferably containing at least 40% by volume thymol), horsemint oil (*Monarda punctata*) (preferably containing at least 40% by volume thymol); oil of lemongrass (preferably containing at least 50% by volume citral; oil of lemon verbena (*Aloysia triphylla*) (preferably containing at least 30% by volume citral); oil of juniper (*juniperus officinalis*) (preferably containing at least 5% by volume T4ol); cypress oil (*Cupressus spp. leylandii*, preferably) (preferably containing at least 5% by volume T4ol); geranium oil (*Pelargonium graveolens,* preferably) (preferably containing at least 60% by volume geraniol and/or linalool).

In the mixture of actives, the following terpenoids are present in the specified ranges:
T4ol 20 to 90%, preferably 30 to 50%;
Linalool 0 to 50% preferably 1 to 20%;
Geraniol 0 to 50% preferably 1 to 20%;
Citral 0 to 50% preferably 1 to 20 %;
Limonene at least 1%, preferably 5 to 20%;
1,8-cineole up to 5%, preferably no more than 2%, for instance 1% or less;
Citral up to 50% preferably 1 to 20%;
Perillaldehyde up to 50%, preferably less than 20%, more preferably less than 10%, for instance 0.01 to 5%;
Menthol less than 50%, preferably less than 20%, more preferably less than 10%, for instance 0.01 to 5%;
Nerolidol preferably up to 50%, more preferably 1 to 20%;
Geraniol up to 50%, preferably 1 to 20%.

Most preferably the mixture of actives comprises tea tree oil in combination with one or more further essential oils, especially lavender, eucalyptus and/or cypress.

According to a further aspect of the invention there is provided a new use of a mixture of essential oils in the manufacture of a composition for use in a method of treatment or prophylaxis of headlice infestation of a human or animal in which the essential oil mixture comprises at least two essential oils, and containing T4ol in an amount of at least 5% by volume.

In this aspect of the invention there is also provided a method of treatment of a human or animal by headlice infestation application of a composition comprising the essential oil mixture containing at least 5% T4ol. The content of T4ol in this aspect is preferably at least 10%, more preferably at least 20%. The mixture is preferably as specified for the first aspect of the invention above.

In the method of treatment in the present invention the composition kills both adult lice and lice eggs.

The carrier may be a liquid, gel, lotion or cream based carrier. The composition may be for application to the head and hair, dry or, more preferably, after pre-wetting, for instance similar to the application of a shampoo. The composition is generally suitable for retaining in contact with the head or hair for a period of at least one half minute, more preferably a period in the range 2 to 500 minutes, for instance in the range 5 to 30 minutes. The composition is preferably removable by water, optionally in conjunction with a supplementary shampoo composition. Preferred carriers are, for instance, oils with which the actives are substantially wholly miscible, or, preferably, aqueous based, whereby the mixture of actives is in a form of a dispersed phase. The dispersed phase may be stabilized by the incorporation of surfactants such as emulsifiers or stabilizers, or by thickening the continuous phase using conventional thickeners.

The total proportion of actives in the composition is preferably in the range 2 to 50%, more preferably 5 to 30%, most preferably 7.5 to 20%.

The carrier may include perfumes (although the components used in the mixture of actives herein generally have pleasant odors without requiring addition of supplementary perfumes), colourants, opacifiers, conditioning agents, moisturisers, surfactants ....

The present compositions are formulated on the basis of their pediculicidal and ovicidal activity against headlice. The use of the preferred components provides consumer acceptable compositions having pleasant odors with a few side effects or contraindications. The compositions have a wide safety margin for use, whilst the volatility of the actives limits patient exposure time and prevents the persistence of residues which could otherwise induce pediculicide resistance.

The following examples illustrate the invention.

### Methods

### Pediculicidal activity test

Using a method developed from an established test for insecticidal activity (WHO/VBC/81.808 (1981)), involving the impregnation of 9 cm filter-papers with 0.6 ml of dissolved sample, the essential oils and terpenoids listed in the results table were tested for their pediculicidal activity. These compounds were diluted to 10% (weight or volume by volume in an appropriate solvent of either absolute ethanol or diethyl ether), and used to impregnate the filter-paper which was subsequently dried for 5 mins to remove solvent. Two controls were run each time, one contained paper which had been impregnated with solvent alone and subsequently evaporated, the other contained paper which remained untreated. The essential oils and terpenoids were ranked according to efficacy, those showing no effect on lice at this concentration were unranked.

10 lice were used in each test replicates, with a target of 5 replicates per concentration of test sample.

Combinations of some oils ere also tested, using 0.3 ml of each of two 10% solutions which were combined prior to impregnation of filter-paper.

A dose-response study was done for (+)-terpinen-4-ol, on the basis of its superior performance in the pediculicidal assay. The method above was repeated using a range of concentrations, from 3% v/v to 10% v/v.

### Ovicidal activity test

The method used for testing ovicidal activity of the compounds and mixtures was based on a method used by MEC Ltd. and involves dipping eggs (which were laid on gauze) in a dilute solution of essential oil or terpenoid specified in the results table for 10 minutes, drying and incubating until the nymphs hatch and die. The percentage mortality was calculated, classing half-hatched and unhatched nymphs as fatalities. In the first stage 10% solutions were used, the most effective terpenoids from the 10% stage were then screened at 5%, the most effective from this stage were screened at 2% and 1%. A minimum of 300 eggs were used in each replicate (the maximum counted being 500), with a target of 5 replicates per concentration of test sample.

### Results

### Terpenoids - Pediculicidal activity

The terpenoids were ranked as follows, they were first arranged on the basis of % kill after 2 hours, upon this is superimposed the time taken to cause the first fatality and, subsequently, the % of dead lice at this time point. Taken together in that order, these three criteria provide a method of separating each terpenoid into a distinct rank in a way which is appropriate to the objective of this investigation i.e. to find the most effective lice killing agent, which works fastest to kill most lice. The results are shown in table 1

**Table 1**

| Rank | | Mean % kill after 3 hr exposure to sample | Time of first fatality and the % mortality achieved at this time point |
|---|---|---|---|
| 1st | (+)-Terpinen-4-ol | 100% | 20% at 15 min (100% at 60 min) |
| 2nd | (-)-Terpinen-4-ol | 100% | 2% at 15 min (74% at 60 min) |
| 3rd | Thymol | 98% | 18% at 30 min |
| 4th | Carvacrol | 98% | 2% at 30 min |
| 5th | Menthone | 96% | 2% at 15 min |
| 6th | Alpha-terpineol | 96% | 8% at 30 min |
| 7th | Linalool | 96% | 2% at 30 min |
| 8th | Perillaldehyde | 90% | 2% at 30 min |
| 9th | Geraniol | 80-85 ? % | 2-4 % at 30 min? |
| 10th | Menthol | 74% | 2% at 45 min |
| 11th | Carveol | 68% | 6% at 45 min |
| 12th | Citral | 66% | 4% at 30 min |
| 13th | Geranyl acetate | 226% | 4% at 1 hr 15 min |
| 14th | Linalyl acetate | 24% | 2% at 2 hr |
| 15th | Cineole | 2% | 2% at 30 min |

### Essential oils - Pediculicidal activity

The rankings for essential oils and mixtures are shown in table 2.

**Table 2**

| Rank | | Mean % kill after 3 hr exposure to sample | Time of first fatality and the % mortality achieved at this time point |
|---|---|---|---|
| 1st | Tea tree | 90% | 10% at 30 min |
| 2nd | Tea tree + lavender | 78% | 4% at 60 min |
| 3rd | Cypress leaf oil | 56% | 10% at 60 min |
| 4th | Tea tree + eucalyptus | 50% | 12% at 45 min |
| 5th | Lavender | 50% | 8% at 45 min |

There were no fatalities in either control group on group on any occasion.
N = 5 for each oil.

The essential oils/mixtures have the following compositions:-
Tea tree: 40% T-4-ol; 5% 1,8-cineole; 1-5% α-terpineol; 15-20% γ-terpinene.
Tea tree + lavender: 20% T-4-ol; 1-5%, 1,8-cineole; 1-5% α-terpineol; 7-10% γ-terpinene.
Cypress leaf: 5-10% T-4-ol; 5-10% α-terpineol; 2-5% γ-terpinene; others mainly pinenes.
Teat tree + eucalyptus: 20% T-4-ol; 30% 1,8-cineole; 1-5% α-terpineol; 15-20% γ-terpinene.
Lavender: 20% linalool; 5% T-4-ol; 1-5% 1,8-cineole; 1-5% α-terpineol; 1-5% γ-terpinene, 20-40% linalyl acetate/lavandulyl acetate.

### Dose-response on Pediculicidal - T4ol

The results of the tests on the dose response of (+)-T4ol are shown in table 3.

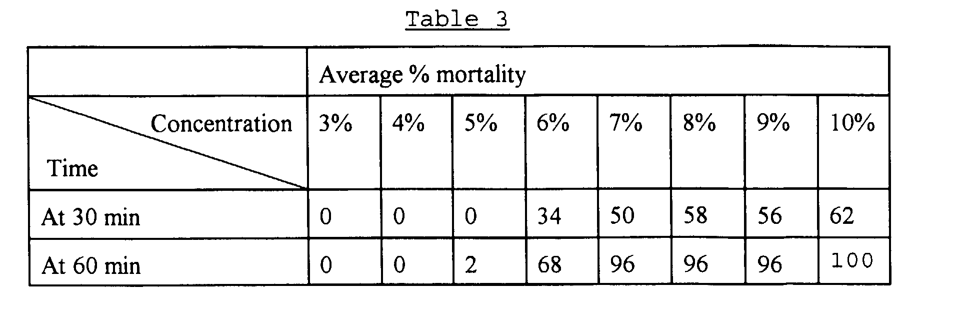

### Ovicidal Activity - Essential oils

At 10% v/v, the following essential oil caused a total louse egg mortality of over 50%.

Geranium oil (68%)

At 10% v/v, the following essential oils caused a total louse egg mortality of less than 50%:

Cypress cone oil, cypress leaf oil, eucalyptus oil, juniper oil, lavender oil, lemon oil, tea tree oil.

The above was based on one or two replicates of the method, described in the previous section, for each oil. Where two replicates have been done the average is shown.

### Ovicidal activity - terpenoids

a) At 10% v/v or w/v. the following terpenoids caused a louse egg mortality of over 50%:

| | |
|---|---|
| Carveol (76%) | Nerolidol (67%) |
| Citral (65%) | (+)-Terpinen-4-ol (70%) |
| Citronellic acid (57%) | (-)-Terpinen-4-ol (61%) |
| | Alpha-terpineol (69%) |
| Linalool (63%) | Thymol |

At 10% v/v or w/v, the following terpenoids caused a louse egg mortality of less than 50%:

| | |
|---|---|
| Camphene | Menth-6-ene-2, 8-diol |
| Camphor | Menthone |
| Carvacrol | Myrcene |
| Cineole | Perillaldehyde |
| Geranyl acetate | Alpha-pinene |
| Limonene | β-pinene |
| Linalyl acetate | Alpha-terpinene |
| Menthane-3,8-diol | |

The above was based on one or two replicates of the method for each oil. Where two replicates have been done the average is shown.
b) At 5% v/v or w/v, the following terpenoids caused a louse egg mortality of over 50%:
Carveol (98%, 54%, 97%, 52%)
- unadjusted terpenoid results were consistent. Where the adjusted mortality (attributable to terpenoid alone i.e. minus the control results) was low, the corresponding control mortality was particularly high. This suggests that adjusted terpenoid mortality can be affected by occasional (and apparently normal) deviations in the control mortality.
Geraniol (82%, 58%, 93%)
- unadjusted terpenoid results were consistent. Where the mortality attributable to terpenoid was low, the corresponding control mortality was particularly high.
Menthol (71%, 80%, 92%, 95%)
- the unadjusted terpenoid results were variable i.e. the variability of the adjusted mortality did not concide with any variability in the control group.
Nerolidol (95%, 54%, 89%, 52%)
- unadjusted terpenoid results were consistent. Where the mortality attributable to terpenoid was low, the corresponding control mortality was particularly high.
Alpha-terpineol (95%, 80%)
- unadjusted terpenoid results were consistent.
Thymol (98%, 80%)
- unadjusted terpenoid results were consistent.

At 5% v/v or w/v, the following terpenoids caused a louse egg mortality of less than 50%:
Citral (80%, 54%, 71%, 10%)
- the variability seen in the adjusted mortality figures partly concided with variation in the terpenoid results and partly with variation in the control results.
Citronellic acid (16%, 51%, 74%, 69%)
- the variability seen in the adjusted mortality figures partly concided with variation in the terpenoid results and partly with variation in the control results.
Linalool (0%, 11%, 9%, 3%, 30%)
- the terpenoid results themselves were variable i.e. the variability of the adjusted mortality did not coincide with any variability in the control group.
(+)-Terpinen-4-ol (14%, 85%, 13%)
- the variability seen in the adjusted mortality figures partly concided with variation in the terpenoid results and partly with variation in the control results.
(-)-Terpinen -4-ol (36%, 6%, 17%, 92, 14%)
- the terpenoid results themselves were variable.

c) At 2% concentrations, the following mortalities were obtained:
Carveol - 3 replicates gave 83%, 92% and 38% (average of 71%).
The terpenoid results themselves were variable i.e. the variability of the adjusted mortality did not coincide with any variability in the control group.
Geraniol - 3 replicates gave 84%, 93% and 63% average of 80%).
The terpenoid results themselves were variable i.e. the variability of the adjusted mortality did not coincide with any variability in the control group.
Menthol - 1 replicate gave 2%.
Nerolidol - 3 replicates gave 84%, 93% and 78% (average of 85%).
Unadjusted terpenoid results were consistent.
Alpha-terpineol - 1 replicate gave 20%.
Thymol - 1 replicate gave 91%.
At 1% concentrations, the results were:
Carveol - 3 replicates gave 55%, 17%% and 3% (average of 25%).
The terpenoid results themselves were variable i.e. the variability of the adjusted mortality did not coincide with any variability in the control group.
Geraniol - 3 replicates gave 43%, 31% and 74% (average of 49%).
The terpenoid results themselves were variable i.e. the variability of the adjusted mortality did not coincide with any variability in the control group.
Menthol - 1 replicate gave 6%.
Nerolidol - 3 replicates gave 82%, 93% and 40% (average of 49%).
The terpenoid results themselves were variable i.e. the variability of the adjusted mortality did not coincide with any variability in the control group.
Alpha-terpineol - 1 replicate gave 2%.
Thymol - 1 replicate gave 56%.

## Claims

1. A composition for application direct to the skin or hair of a human for the treatment or prophylaxis of headlice infestation comprises a carrier and a mixture of actives, in which the mixture of actives comprises at least 20% by volume terpinene-4-ol (T4ol), no more than 5% 1, 8-cineole and at least 35% by volume components selected from T4ol, thymol, alpha-terpineol, linalool and carveol.

2. A composition according to claim 1 in which T4ol is present in the actives in an amount in the range 30 to 50%.

3. A composition according to claim 1 or 2 in which 1,8-cineole is present in an amount up to 2% by weight.

4. A composition according to claim 1 or 2 in which the ratio of T4ol to cineole at least 5:1, preferably at least 10:1, more preferably at least 20:1.

5. A composition according to any preceding claim in which the mixture of actives comprises,
T4ol 20 to 90%, preferably 30 to 50%;
Linalool 0 to 50% preferably 1 to 20%;
Geraniol 0 to 50% preferably 1 to 20%;
Citral 0 to 50% preferably 1 to 20 %;
Limonene at least 1%, preferably 5 to 20%;
1,8-cineole up to 5%, preferably no more than 2%, for instance 1% or less;
Citral up to 50% preferably 1 to 20%;
Perillaldehyde up to 50%, preferably less than 20%, more preferably less than 10%, for instance 0.01 to 5%;
Menthol less than 50%, preferably less than 20%, more preferably less than 10%, for instance 0.01 to 5%;
Nerolidol preferably up to 50%, more preferably 1 to 20%;
Geraniol up to 50%, preferably 1 to 20%.

6. A composition according to any preceding claim which is a liquid, preferably comprising a cosmetically-acceptable carrier.

7. A composition according to any preceding claim in which the mixture of actives comprises essential oils, preferably selected from tea tree oil, thyme oil, peppermint oil, lavender oil, cabreuva or cubeb seed oil, oil of ajowan, lemongrass oil, lemon verbena oil, juniper oil, cypress oil, geranium oil and mixtures.

8. A composition according to claim 7 in which the carrier is aqueous and preferably comprises surfactant, the composition preferably being a shampoo.

9. A composition according to any preceding claim in which the total volume of actives in the composition is in the range 2 to 30%, preferably 5 to 30%, most preferably 7.5 to 20%.

10. Use of a mixture of actives comprising at least 20% by volume terpinene-4-ol (T4ol), no more than 5% 1, 8-cineole containing at least 35% by volume components selected from T4ol thymol, alpha-terpineol, linalool and carveol, in the manufacture of a composition for use in a method of treatment or prophylaxis of a human or animal.

11. Use according to claim 10 in which the said method is for the treatment or prophylaxis of headlice infestation.

12. Method of killing headlice and/or their eggs comprising applying to a location where the eggs or lice are present or believed to be present a composition according to any of claims 1 to 9.

13. Method of preventing headlice infestation by applying to a location which could come or has come into contact with headlice or their eggs a composition according to any of claims 1 to 9.

14. Method according to claim 12 or claim 13 is which the location is the head and hair of a human.

15. Method according to any of claims 12 to 14 in which the composition remains in contact with the hair for a period in the range 0.5 to 500 minutes, preferably 2 to 30 minutes.

16. A method according to claim 15 in which the composition is removed by rinsing with water only.

17. Use of a mixture of essential oils in the manufacture of a composition for use in a method of treatment or prophylaxis of headlice infestation of a human or animal in which the essential oil mixture comprises at least two essential oils, and containing T4ol in an amount of at least 5% by volume.

18. Method of treatment of a human or animal headlice infestation by application of a composition comprising the essential oil mixture defined in claim 17.
